# EUROPEAN PATENT SPECIFICATION

(11) **EP 0 612 519 B1**
(45) Date of publication and mention of the grant of the patent: **04.04.2001**
(21) Application number: 94102012.5
(22) Date of filing: 09.02.1994
(51) Int. Cl.: A61K 9/02, A61K 31/19

(54) **Use of a calcium formulation in a medicament for therapeutic application to milk cows suffering from hypocalcaemia**
Die Verwendung eines Calciumpräparates in einem Medikament zur Behandlung von an Hypocalcämie leidenden Milchkühen
Utilisation d'une formulation de calcium dans une médicament pour traiter des vaches à lait atteintes de l'hypocalcémie

(30) Priority: 23.02.1993 DE 4305469
(43) Date of publication of application: 31.08.1994
(73) Proprietor: Werner, Wolfgang Dr., D-57258 Freudenberg (DE); Sirkkola, Heikki, SF-13100 Hämeelinna 10 (FI)
(72) Inventor: Werner, Wolfgang Dr., D-57258 Freudenberg (DE); Sirkkola, Heikki, SF-13100 Hämeelinna 10 (FI)
(74) Representative: Grosse, Wolf-Dietrich Dipl.-Ing., Patentanwälte Hemmerich & Partner

(56) References cited:
- EP-A- 0 335 140
- EP-A- 0 390 541
- DE-A- 2 301 896
- DE-C- 812 812
- DE-C- 814 770
- DE-C- 830 826
- DE-C- 855 896
- GB-A- 2 140 687

## Description

The invention refers to a use of a calcium formulation for the preparation of medicaments for therapeutic application to milk cows post partum and/or sub partum, suffering from hypocalcaemia.

It has become apparent that approximately five to ten percent of milk cows suffer from hypocalcaemia. In the meaning of this present, the term "milk cows" refers to post partum and sub partum cows. Considerable amounts of calcium are yielded with the milk. The body cannot adapt fast enough and so, substantial losses of calcium arise the are not, or only inadequately, replaced. A severely reduced calcium level is so generated and the metabolism of the muscles is also acutely affected. A cow suffering from hypocalcaemia can, for instance, no longer stand up and, in critical cases, hypocalcaemia may even prove lethal.

A customary method for treating hypocalcaemia is the application of calcium, or of solutions containing calcium and magnesium by intravenous injection. An intravenous injection requires to be made by a veterinary surgeon and the treatment hence, involves trouble and is expensive. Efforts have therefore been made to apply calcium and calcium/magnesium formulations orally. This, however, has also proved to be problematic. If even a few drops only of the formulation enter the trachea, this will cause pulmonary necrosis. In addition, it has been found that the effectiveness of such treatment is impaired in the case of ruminants whose reaction to the calcium will be insufficient and utilization of the formulation so applied is hence, inadequate. And, depending on the shape and preparation of the calcium physic applied, considerable necroses may be generated in the ruminant's stomach.

According to an article published in 1990 in "Dairy Science" (vol. 73, No. 2, pages 470 to 474), in cases of hypomagnesia, magnesium formulations are injected as an aqueous solution either intravenously, or subcutaneously or administered orally or through the rectum. Apart from the disadvantages of injections and the hazard of oral application that have already been referred to, the additional disadvantage arising in this case is the hardly noticeable and, at least partial, seeping of the physic from the animal's mouth, or anus.

The object of this invention is to prepare a medicament containing calcium formulation for treating hypocalcaemia in cows such that the owner of the animal will be in a position to administer it on his own without having to call in a veterinary surgeon and such that any hazards resulting from inappropriate application of the compound will be avoided.

This object is achieved by embedding the calcium formulation in a suppository. The object is likewise achieved by including the calcium formulation in a pasty substance or a gel that is also introduced through the rectum of a cow. The farmer is well able to enter the suppositories or pasty substance into the rectum on his own. One mistake only can be made in this case in that the physic is not entered far enough into the intestine so causing the substance to be discharged prematurely, no further hazards need be expected in context with a rectal application.

The features of the invention are explained by means of examples.

In the first place, a calcium formulation, or as the case may be, a calcium/magnesium formulation must be determined that will not adversely affect the mucous membranes of the rectum. Pure calcium and even the latter's basic hydroxides must hence, be ruled out, whereas calcium gluconate, calcium sucrate and the calcium and/or calcium/magnesium salts of heptagluconic acid, lactic acid, etc. may be taken into positive consideration. These substances can be embedded in media that will dissolve in the rectum, so for instance in waxes, cacao-nut oil and similar matter. The calcium and/or calcium/magnesium formulations may be sealed in the wax in form of a substance, or a solution. However, there also exists the possibility of shaping the suppositories such that they have more than one single cavity, i.e. a number of smaller cavities for accommodating the calcium and/or calcium/magnesium formulation or solutions of the latter in.

The introduction of the suppository is facilitated by its customary cone shape. The peristaltic of the alimentary canal and/or other motions of the latter will then advance the ingress of the suppository into the rectum range by engaging the "cone base", whilst the tip of the suppository offers practically very little resistance.

For administering the formulation it will therefore suffice to enter the suppository into the rectum; the peristaltic and other motions of the alimentary canal will then cause the suppository to be conveyed onward. Decomposition by thermal influences and/or break-up of the suppository body, as such, is caused by the melting process, and the compound so introduced will be absorbed by the mucous membranes of the rectum without harming it and be transferred to the blood-stream.

On the other hand, the formulation need not necessarily be introduced by means of a suppository in which it is contained: the calcium and/or calcium/magnesium formulation may just as well be administered in the form of a pasty or a gel-type substance from a tube, for instance. Administering the active substance by this means is also fairly unproblematic. A plastic tubular extension (of the known type) can, for in-stance, be screwed onto the tube neck for entering into the rectum. The formulation is then administered by squeezing the tube and so causing the pasty substance or gel to issue through opening in the extension attached to the tube. In this case also, the substance contained in the formulation can be introduced far enough into the rectum and, its pasty consistency will prevent the carrier substance from simply seeping out, a process that is hardly noticeable in the case of aqueous solutions. Nevertheless, if the cow were to defecate directly, or just shortly after the preparation has been administered, the dose must then be considered to be lost, and application of the formulation should be repeated, same as in the case of defecation immediately after the introduction of a suppository, where the possibility of administering a second suppository, if necessary, always exists.

The peristaltic of the intestine and also the other waves of the bowels generated by the animal's movements have been found to be particularly helpful when using suppositories as they will have the effect of automatically drawing the latter deeper into the bowels. On the other hand, in the case of severe hypocalcaemia that has advanced to a critical stage, the peristaltic will also fail in line with the animal's immobilisation. In this case, the application of a suppository that is introduced at a full arm's length (approx. 80 cms) so that it will not be prematurely ejected, can safely ensure the effects of the formulation.

According to the doctrine of the invention, the calcium level of a sick animal can so be raised without any problems.

## Claims

1. Use of a calcium formulation for the preparation of medicaments for therapeutic application to milk cows post partum and/or sub partum suffering from hypocalcaemia, wherein said calcium formulation is contained in a suppository.

2. Use of a calcium formulation as in claim 1 wherein said calcium formulation is contained in a pasty substance intended for administering by way of the rectum of the cow.

## Patentansprüche

1. Verwendung eines Calciumpräparats für die Herstellung von Medikamenten zur therapeutischen Anwendung bei an Hypocalcämie leidenden Milchkühen post partum und/oder sub partum, wobei das Calciumpräparat in einem Zäpfchen enthalten ist.

2. Verwendung eines Calciumpräparats nach Anspruch 1, wobei das Calciumpräparat in einer pastenförmigen Substanz enthalten ist, welche zur Verabreichung über das Rektum der Kuh bestimmt ist.

## Revendications

1. Utilisation d'une formulation de calcium en vue de la préparation de médicaments pour application thérapeutique à des vaches laitières post partum et/ou sub partum souffrant d'hypocalcémie, ladite formulation de calcium étant contenue dans un suppositoire.

2. Utilisation d'une formulation de calcium selon la revendication 1, la formulation de calcium se trouvant dans une substance pâteuse destinée à être administrée par le rectum de la vache.
